# EUROPEAN PATENT SPECIFICATION

(11) **EP 4 456 080 B1**
(45) Date of publication and mention of the grant of the patent: **24.06.2026**
(21) Application number: 24172068.9
(22) Date of filing: 24.04.2024
(51) Int. Cl.: G16H 20/40, G16H 30/40, G16H 15/00, G16H 40/60, G16H 80/00

(54) **DEVICE FOR SETTING COORDINATES FOR EACH PART OF ORGAN OF HUMAN BODY AND METHOD OF OPERATING THE SAME**
VORRICHTUNG ZUR KOORDINATENEINSTELLUNG FÜR JEDEN TEIL EINES MENSCHLICHEN KÖRPERS UND BETRIEBSVERFAHREN DAFÜR
DISPOSITIF DE RÉGLAGE DE COORDONNÉES POUR CHAQUE PARTIE D'ORGANE DU CORPS HUMAIN ET SON PROCÉDÉ DE FONCTIONNEMENT

(30) Priority: 24.04.2023 KR 20230053140
(43) Date of publication of application: 30.10.2024
(73) Proprietor: Hutom Inc., Seoul 04151 (KR)
(72) Inventor: PARK, Seok Rae, 14244 Gwangmyeong-si (KR); KIM, Keun Young, 04168 Seoul (KR); JUNG, Kyung Yoon, 04171 Seoul (KR); PARK, Bo Kyoung, 04086 Seoul (KR)
(74) Representative: dompatent

(56) References cited:
- US-A1- 2013 229 432
- US-A1- 2019 347 791
- US-A1- 2022 067 925
- PAPENKORT STEFAN ET AL: "A geometry model of the porcine stomach featuring mucosa and muscle layer thicknesses", JOURNAL OF THE MECHANICAL BEHAVIOR OF BIOMEDICAL MATERIALS, vol. 142, 15 April 2023 (2023-04-15), AMSTERDAM, NL, pages 105801, XP093201270, ISSN: 1751-6161, DOI: 10.1016/j.jmbbm.2023.105801

## Description

### [TECHNICAL FIELD]

Embodiments of the present disclosure described herein relate to an electronic device, and more particularly, relate to a device for setting coordinates of each part of an organ of a human body and a method of operating the same.

### [BACKGROUND ART]

To accurately describe a surgical operation, it is required to specify an exact position of an organ of each patient. There are names that specify organ parts, but since the corresponding organ parts correspond to a wide range, it may be difficult to specifically and accurately specify a position within the corresponding organ part. In detail, since there are differences in a feature of an organ shape or a size and shape of the organ for each patient, it is difficult to accurately describe a position of the organ part in a specific manner using general coordinates systems such as a rectangular coordinate system and a spherical coordinate system. Thus, it is required to designate the position in consideration of the feature of the organ shape or the shape or the like of the organ of each patient.

A similar device is disclosed in US20190347791A1 disclosing method for analyzing a skeletal medical imaging recording. In an embodiment, the method includes providing a skeletal medical imaging recording; automatically determining reference points in the image recording; and calculating an orthopedic result value based at least upon a spacing between two reference points and/or upon an angle defined by reference points.

Another similar method is known from US2022067925A1 disclosing a method calculating a medial-axis tree graph of a volume of an organ of a patient in a computerized anatomical map of the volume. A predefined number of major branches in the tree graph are identified. Using the identified major branches, one or more known anatomical opening regions of the volume are identified in the anatomical map.

### [DETAILED DESCRIPTION OF THE INVENTION]

### [TECHINICAL PROBLEM]

Embodiments of the present disclosure provide a device that may describe an exact position of an organ of a human body through an isoline in surgery description.

### [TECHNICAL SOLUTION]

According to an embodiment, a device for setting coordinates of each part of an organ of a human body includes a memory, and a processor that receives organ information on the organ, sets a plurality of isolines connecting a first area corresponding to a first part constituting the organ and a second area corresponding to a second part constituting the organ, and generates organ coordinate system information including isoline information representing the plurality of isolines and the organ information.

According to an embodiment, a method of operating a device for setting coordinates of each part of an organ of a human body includes receiving organ information on the organ, setting a plurality of isolines connecting a first area corresponding to a first part constituting the organ and a second area corresponding to a second part constituting the organ, and generating organ coordinate system information including the organ information and isoline information on the plurality of isolines.

### [ ADVANTAGEOUS EFFECTS OF THE INVENTION]

According to the above description, in surgical description, an exact position of an organ of a human body may be described through an isoline.

### [ DESCRIPTION OF THE DRAWINGS]

FIG. 1 is a block diagram of a device according to the present disclosure;
FIG. 2 is a flowchart for describing a method of operating the device according to the present disclosure;
FIG. 3 is an exemplary view illustrating organ information according to the present disclosure; and
FIG. 4 is a view for describing a method of setting a plurality of isolines according to the present disclosure.

### [ BEST MODE]

Throughout the present disclosure, the same reference numerals refer to the same components. The present disclosure does not describe all components of embodiments, and general contents or duplicated contents between the embodiments in the technical field to which the present disclosure pertains will be omitted. Terms "unit, module, member, and block" used in the specification may be implemented as software or hardware, and according to embodiments, a plurality of "units, modules, members, and blocks" may be implemented as a single component or one "unit, module, member, and block" may include a plurality of components.

Throughout the specification, when it is described that a first component is "connected" to a second component, this includes not only a case in which the first component is directly connected to the second component but also a case in which the first component is indirectly connected to the second component, and the indirect connection includes connection through a wireless communication network.

Further, when a part "includes" a component, this means that a third component is not excluded but may be further included unless otherwise stated.

Throughout the specification, when a first member is located "on" a second member, this case includes not only a case in which the first member is in contact with the second member but also a case in which a third member is present between the two members.

Terms such as first and second are used to distinguish a first component from a second component, and the components are not limited by the above-described terms.

Singular expressions include plural expressions unless clearly otherwise indicated in the context.

In each of operations, an identification code is used for convenience of description and does not describe a sequence of the operations, and the operations may be performed in a different order from the specified order unless the context clearly states a specific order.

Hereinafter, the operating principles and embodiments of the present disclosure will be described with reference to the accompanying drawings.

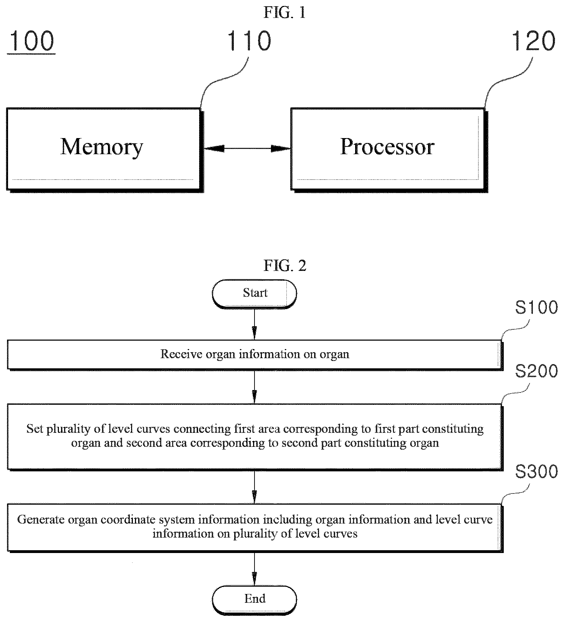

FIG. 1 is a block diagram of a device 100 according to the present disclosure.

Referring to FIG. 1, the device 100 is a device that divides each organ area to form an isoline to specify a position of an organ during surgery and provide organ coordinate system information so that a user may describe an exact position of the organ through the isoline.

The device 100 includes a memory 110 and a processor 120.

The memory 110 may store data about an algorithm for controlling operations of components inside the device and a program that reproduces the algorithm, and at least one processor 120 that performs the above-described operations using the data stored in the memory 110 may be implemented. Here, the memory 110 and the processor 120 may be implemented as separate chips. Further, the memory 110 and processor 120 may be implemented as a single chip.

The memory 110 may store data for supporting various functions of the device and a program for an operation of the processor 120, may store input/output data, and may store a number of application programs (or applications) running on the device, data for an operation of the device, and commands. At least some of these application programs may be downloaded from an external server via wireless communication.

The memory 110 may include at least one storage medium among a flash memory type memory, a hard disk type memory, a solid state disk (SSD) type memory, a silicon disk drive (SDD) type memory, a multimedia card micro type memory, a card type memory (e.g., an SD or XD memory or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. Further, the memory 110 is separate from the device, but may be a database connected by wire or wirelessly.

The processor 120 receives organ information on an organ. The processor 120 sets a plurality of isolines connecting a first area corresponding to a first part constituting the organ and a second area corresponding to a second part constituting the organ. The processor 120 generates organ coordinate system information including isoline information representing the plurality of isolines sand the organ information.

Although not illustrated, the device 100 may further include a communication unit capable of communicating with an external unit. In this case, the communication unit may include a wireless communication module that supports various wireless communication methods such as global system for mobile communication (GSM), code division multiple access (CDMA), wideband code division multiple access (WCDMA), an universal mobile telecommunications system (UMTS), time division multiple access (TDMA), a long term evolution (LTE), a fourth generation mobile communication (4G), a fifth generation mobile communication (5G), and a sixth generation mobile communication (6G) in addition to a Wi-Fi module and a wireless broadband module.

According to the above description, in surgical description, the exact position of the organ of a human body may be described through the isolines.

FIG. 2 is a flowchart for describing a method of operating the device according to the present disclosure.

Referring to FIG. 2, the method of operating the device according to the present disclosure includes operation S100, operation S200, and operation S300.

In operation S100, an operation of receiving the organ information on the organ is performed.

In operation S200, an operation of setting the plurality of isolines connecting the first area corresponding to the first part constituting the organ and the second area corresponding to the second part constituting the organ is performed.

In operation S300, an operation of generating the organ coordinate system information including the organ information and the isoline information on the plurality of isolines.

According to the above description, in surgical description, the exact position of the organ of the human body may be described through the isolines.

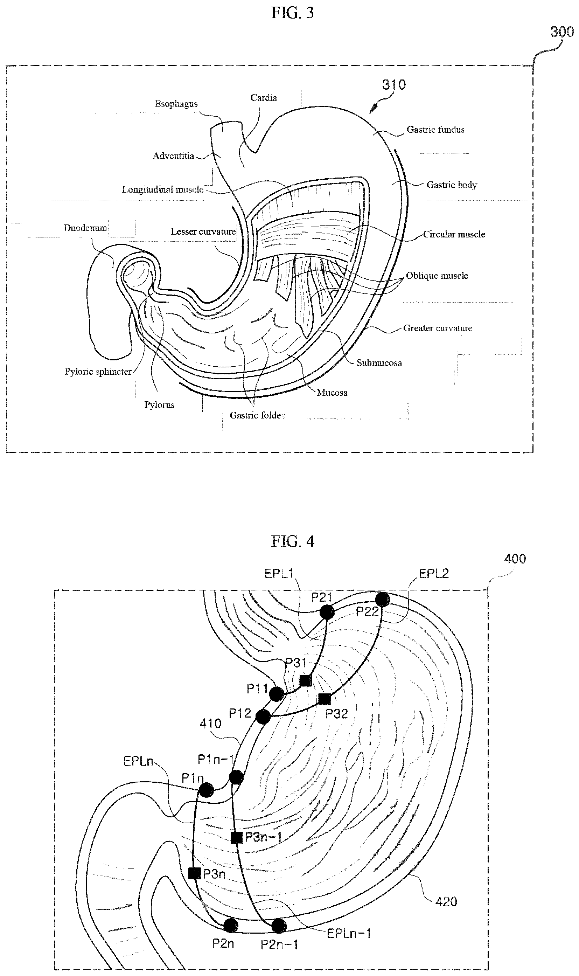

FIG. 3 is an exemplary view illustrating organ information according to the present disclosure.

Referring to FIG. 3, the organ information includes an organ image 300 representing an organ 310. The organ information may include a name of the organ 310 and medical names of respective portions constituting the organ 310.

Referring to FIG. 3, for example, the organ 310 may correspond to a stomach. The name of the organ 310 may be stomach. The medical names of the respective portions of the stomach may include esophagus, adventitia, cardia, longitudinal muscle, lesser curvature, gastric fundus, gastric body, circular muscle, oblique muscle, greater curvature, gastric folds, submucosa, mucosa, pylorus, pyloric sphincter, and the like.

In some embodiments, the first part for setting the isoline may correspond to a first medical name, and the second part may correspond to a second medical name. Referring to FIG. 3, for example, the first medical name may be the lesser curvature, and the first part may be a part representing the lesser curvature. The second medical name is the greater curvature and the second part may be a part representing the greater curvature. However, the present disclosure is not limited thereto.

FIG. 4 is a view for describing a method of setting a plurality of isolines according to the present disclosure.

Referring to FIG. 4, an organ image 400 of FIG. 4 may be an image representing a cross-sectional view of the organ. Referring to FIG. 4, for example, the organ image 400 may include a cross-sectional view of the stomach. However, the present disclosure is not limited thereto, and the organ image 400 may represent a three-dimensional organ which is like the organ image 300 of FIG. 3.

In some embodiments, a first area 410 may correspond to the first part, and the first part may be the lesser curvature. A second area 420 may correspond to the second part, and the second part may be the greater curvature.

The processor 120 sets a plurality of first points P11, P12, P1n-1, and P1n on the first area 410. The number of the plurality of first points P11, P12, P1n-1, and P1n may be n. n may be an integer of 2 or more. The processor 120 **sets** a plurality of second points P21, P22, P2n-1, and P2n on the second area 420. The number of the plurality of second points P21, P22, P2n-1, and P2n may be n. The processor 120 sets the plurality of isolines EPL1, EPL2, EPLn-1, and EPLn by connecting the first points and the second points positioned at the same order from the starting points to the end points of the first area 410 and the second area 420. For example, the starting point of the first area 410 may be the first point P11, and the end point of the first area 410 may be the first point P1n. For example, the starting point of the second area 420 may be the second point P21, and the end point of the second area 420 may be the second point P2n. For example, the first point and the second point positioned at a first order may be the first point P11 and the second point P21, respectively. As another example, the first point and the second point positioned at a second order may be the first point P12 and the second point P22, respectively. As still another example, the first point and the second point positioned at a n^{th} order may be the first point P1n and the second point P2n, respectively. The number of the plurality of isolines EPL1, EPL2, EPLn-1, and EPLn may be n.

According to the above description, a position of an organ part may be described using the isoline formed in the divided range. By ordering the formed isolines, the user may describe the exact position using the number of the isoline during surgery. In a describing method, for example, the position of the organ part may be described by the name of the organ, an order of the isoline, a distance from a reference point of an inner line (or a reference point of an outer line) along the isoline, and the like.

The plurality of isolines EPL1, EPL2, EPLn-1, and EPLn may be positioned on an inner circumferential surface corresponding to a periphery of the organ. Since the actual organ has a three-dimensional shape, the plurality of isolines EPL1, EPL2, EPLn-1, and EPLn may have a curved shape positioned along an inner circumferential surface of the three-dimensional organ.

The processor 120 recognizes a detailed area range of each organ and then divide the corresponding range at equal intervals. That is, the processor 120 sets the plurality of first points P11, P12, P1n-1, and P1n at equal intervals. Referring to FIG. 4, for example, a first interval between the plurality of first points P11, P12, P1n-1, and P1n is the same. The processor 120 sets the plurality of second points P21, P22, P2n-1, and P2n at equal intervals. Referring to FIG. 4, for example, a second interval between the plurality of second points P21, P22, P2n-1, and P2n is the same. That is, the processor 120 may specify an inner area (lesser curvature) and an outer area (greater curvature) of the stomach as ranges and may divide the inner area and the outer area into the same number of parts. The size of the lesser curvature and the size of the greater curvature may be different from each other. Therefore, the first interval between the plurality of first points P11, P12, P1n-1, and P1n and the second interval between the plurality of second points P21, P22, P2n-1, and P2n may be different from each other. Referring to FIG. 4, for example, when the size of the lesser curvature is smaller than the size of the greater curvature, the first interval may be smaller than the second interval. The processor 120 sequentially sets a number to each of the plurality of isolines from the isoline formed by connecting the starting points to the isoline formed by connecting the end points. For example, the isoline formed by connecting the starting points is the first isoline EPL1, and the isoline formed by connecting the end points may be the n^{th} isoline EPLn. A number is set to each of the plurality of isolines EPL1, EPL2, EPLn-1, and EPLn sequentially form the first isoline EPL1 to the n^{th} isoline EPLn. In this case, the first isoline EPL1 is set to a first order, the second isoline EPL2 may be set to a second order, and the n^{th} isoline may be set to an n^{th} order.

The processor 120 calculates a separation distance from the first point or the second point positioned at the same order on the isoline formed by connecting the first point and the second point positioned at the same order. For example, a separation distance from the first point P11 or the second point P21 on the first isoline EPL1 formed by connecting the first point P11 and the second point P21 positioned at the first order may be calculated. The processor 120 obtains
a third point positioned at a distance spaced apart from the first point or the second point positioned at the same order. For example, the processor 120 acquires a third point P31 that is spaced apart from the first point P11 (or the second point P21) on the first isoline EPL1. As another example, a third point P32 is obtained on the second isoline EPL2, a third point P3n-1 is obtained on the (n-1)^{th} isoline EPLn-1, and a third point P3n is obtained on the n^{th} isoline EPLn. The processor 120 generates the organ coordinate system information that further includes point information representing the plurality of first points P11, P12, P1n-1, and P1n, the plurality of second points P21, P22, P2n-1, and P2n, and at least one third point. Accordingly, coordinates of the position of the organ part positioned on the isoline may be obtained.

Meanwhile, the disclosed embodiments may be implemented in the form of a recording medium that stores a computer-readable command. The command may be stored in the form of a program code, and when the command is executed by a processor, a program module may be generated to perform operations of the disclosed embodiments. The recording medium may be implemented as a computer-readable recording medium.

The computer-readable recording medium includes all types of recording media storing commands that may be decoded by a computer. Examples of the computer-readable recording medium may include the ROM, the RAM, a magnetic tape, a magnetic disk, a flash memory, an optical data storage device, and the like.

## Claims

1. A device for setting coordinates of each part of an organ of a human body through an isoline in surgery **,** the device comprising:
a memory (110); and
a processor (120) configured to receive organ information on the organ, wherein the organ information includes an organ image (300) representing the organ (310), set a plurality of isolines (EPL1, EPL2, EPLn-1, EPLn) connecting a first area (410) corresponding to a first part constituting the organ and a second area (420) corresponding to a second part constituting the organ, wherein the plurality of isolines (EPL1, EPL2, EPLN- 1, EPLn) are positioned on an inner circumferential surface corresponding to a periphery of the organ, and generate organ coordinate system information including isoline information representing the plurality of isolines (EPL1, EPL2, EPLn-1, EPLn) and the organ information,
wherein the processor (120) is configured to: set a plurality of first points (P11, P12, Pin-1, Pin) on the first area (410); and set a plurality of second points (P21, P22, P2n-1, P2n) on the second area (420), and
wherein the plurality of isolines (EPL1, EPL2, EPLn-1, EPLn) are set by connecting the plurality of first points (P11, P12, Pin-1, Pin) and the plurality of second points (P21, P22, P2n-1, P2n) positioned at the same order from starting points to end points of the first area (410) and the second area (420),
**characterized in that** the processor (120) is configured to: set the plurality of first points (P11, P12, Pin-1, Pin) at equal intervals; set the plurality of second points (P21, P22, P2n-1, P2n) at equal intervals,
wherein a number is set to each of the plurality of isolines (EPL1, EPL2, EPLn-1, EPLN) sequentially from an isoline formed by connecting the starting points to an isoline formed by connecting the end points, and
wherein the processor (120) is further configured to: calculate a distance spaced apart from the plurality of first points (P11, P12, P1n-1, P1n) or the plurality of second points (P21, P22, P2n-1, P2n) positioned at the same order on an isoline formed by connecting the plurality of first points and the plurality of second points positioned at the same order; acquire a plurality of third points (P31, P32, P3n-1, P3n) positioned at the distance spaced apart from the plurality of first points or the plurality of second points positioned at the same order; and generate the organ coordinate system information further including point information representing the plurality of first points (P11, P12, P1n-1, P1n), the plurality of second points (P21, P22, P2n-1, P2n), and the plurality of third points (P31, P32, P3n-1, P3n).

2. The device of claim 1,
wherein the organ information includes a name of the organ (310) and medical names of respective parts constituting the organ (310),
wherein the first part corresponds to a first medical name,
wherein the second part corresponds to a second medical name,
wherein the organ corresponds to a stomach,
wherein the first medical name includes a lesser curvature, and
wherein the second medical name includes a greater curvature.

3. A method of operating a device for setting coordinates of each part of an organ of a human body through an isoline in surgery , the method comprising:
receiving organ information on the organ, wherein the organ information includes an organ image (300) representing the organ (310);
setting a plurality of isolines (EPL1, EPL2, EPLn-1, EPLn) connecting a first area (410) corresponding to a first part constituting the organ and a second area (420) corresponding to a second part constituting the organ, wherein the plurality of isolines (EPL1, EPL2, EPLN- 1, EPLN) are positioned on an inner circumferential surface corresponding to a periphery of the organ; and
generating organ coordinate system information including the organ information and isoline information on the plurality of isolines (EPL1, EPL2, EPLN- 1, EPLN),
wherein the setting of the plurality of isolines includes: setting a plurality of first points (P11, P12, Pin-1, Pin) on the first area (410); setting a plurality of second points (P21, P22, P2n-1, P2n) on the second area (420); and setting the plurality of isoline (EPL1, EPL2, EPLn-1, EPLn) by connecting the plurality of first points (P11, P12, Pin-1, Pin) and the plurality of second points (P21, P22, P2n-1, P2n) positioned at the same order from starting points to end points of the first area (410) and the second area (420),
**characterized in that** the plurality of first points (P11, P12, Pin-1, Pin) are set at equal intervals,
wherein the plurality of second points (P21, P22, P2n-1, P2n) are set at equal intervals,
wherein a number is set to each of the plurality of isolines (EPL1, EPL2, EPLn-1, EPLN) sequentially from an isoline formed by connecting the starting points to an isoline formed by connecting the end points, and
wherein the method further comprises: calculating a distance spaced apart from the plurality of first points (P11, P12, Pin-1, P1n) or the plurality of second points (P21, P22, P2n-1, P2n) positioned at the same order on an isoline formed by connecting the plurality of first points and the plurality of second points positioned at the same order; acquiring a plurality of third points (P31, P32, P3n-1, P3n) positioned at the distance spaced apart from the plurality of first points or the plurality of second points positioned at the same order; and generating the organ coordinate system information further including point information representing the plurality of first points (P11, P12, P1n-1, P1n), the plurality of second points (P21, P22, P2n-1, P2n), and the plurality of third points (P31, P32, P3n-1, P3n).

4. The method of claim 3,
wherein the organ information includes a name of the organ (310) and medical names of respective parts constituting the organ (310),
wherein the first part corresponds to a first medical name,
wherein the second part corresponds to a second medical name,
wherein the organ corresponds to a stomach,
wherein the first medical name includes a lesser curvature, and
wherein the second medical name includes a greater curvature.

5. A computer-readable recording medium coupled to a computer and storing a program for executing the method of any one of claims 3 to 4.

## Patentansprüche

1. Vorrichtung zum Einstellen von Koordinaten jedes Teils eines Organs eines menschlichen Körpers durch eine Isolinie in der Chirurgie, wobei die Vorrichtung aufweist:
einen Speicher (110); und
einen Prozessor (120), der so ausgebildet ist, dass er Organinformationen über das Organ empfängt, wobei die Organinformationen ein Organbild (300) enthalten, welches das Organ (310) darstellt, eine Vielzahl von Isolinien (EPL1, EPL2, EPLn-1, EPLn) festlegt, die einen ersten Bereich (410), der einem das Organ bildenden ersten Teil entspricht, und einen zweiten Bereich (420), der einem das Organ bildenden zweiten Teil entspricht, verbinden, wobei die Vielzahl von Isolinien (EPL1, EPL2, EPLn-1, EPLn) auf einer Innenumfangsfläche positioniert sind, die einer Peripherie des Organs entspricht, und Organ-Koordinatensystem-Informationen erzeugt, die Isolinieninformationen enthalten, welche die Vielzahl von Isolinien (EPL1, EPL2, EPLn-1, EPLn) und die Organinformationen darstellen,
wobei der Prozessor (120) für Folgendes ausgebildet ist: Festlegen einer Vielzahl von ersten Punkten (P11, P12, Pin-1, Pin) auf dem ersten Bereich (410); und Festlegen einer Vielzahl von zweiten Punkten (P21, P22, P2n-1, P2n) auf dem zweiten Bereich (420), und
wobei die Vielzahl von Isolinien (EPL1, EPL2, EPLn-1, EPLn) durch Verbinden der Vielzahl von ersten Punkten (P11, P12, Pin-1, Pin) und der Vielzahl von zweiten Punkten (P21, P22, P2n-1, P2n), die in der gleichen Reihenfolge von Startpunkten zu Endpunkten des ersten Bereichs (410) und des zweiten Bereichs (420) positioniert sind, festgelegt werden,
**dadurch gekennzeichnet, dass** der Prozessor (120) für Folgendes ausgebildet ist: Festlegen der Vielzahl von ersten Punkten (P11, P12, Pin-1, Pin) in gleichen Abständen; Festlegen der Vielzahl von zweiten Punkten (P21, P22, P2n-1, P2n) in gleichen Abständen,
wobei jeder der Vielzahl von Isolinien (EPL1, EPL2, EPLn-1, EPLN) eine Nummer zugeordnet wird, und zwar nacheinander von einer Isolinie, die durch Verbinden der Startpunkte gebildet wird, bis zu einer Isolinie, die durch Verbinden der Endpunkte gebildet wird, und
wobei der Prozessor (120) ferner für Folgendes ausgebildet ist: Berechnen eines Abstands, der von der Vielzahl von ersten Punkten (P11, P12, P1n-1, P1n) oder der Vielzahl von zweiten Punkten (P21, P22, P2n-1, P2n), die in der gleichen Reihenfolge positioniert sind, auf einer Isolinie beabstandet ist, die durch Verbinden der Vielzahl von ersten Punkten und der Vielzahl von zweiten Punkten, die in der gleichen Reihenfolge positioniert sind, gebildet wird; Erfassen einer Vielzahl von dritten Punkten (P31, P32, P3n-1, P3n), die in dem Abstand von der Vielzahl von ersten Punkten oder der Vielzahl von zweiten Punkten, die in der gleichen Reihenfolge positioniert sind, positioniert sind; und Erzeugen der Organ-Koordinatensystem-Informationen, die ferner Punktinformationen enthalten, welche die Vielzahl von ersten Punkten (P11, P12, P1n-1, P1n), die Vielzahl von zweiten Punkten (P21, P22, P2n-1, P2n) und die Vielzahl von dritten Punkten (P31, P32, P3n-1, P3n) darstellen.

2. Vorrichtung nach Anspruch 1, wobei die Organinformationen eine Bezeichnung des Organs (310) und medizinische Bezeichnungen der jeweiligen Teile, aus denen das Organ (310) besteht, enthalten,
wobei der erste Teil einer ersten medizinischen Bezeichnung entspricht,
wobei der zweite Teil einer zweiten medizinischen Bezeichnung entspricht,
wobei das Organ einem Magen entspricht,
wobei die erste medizinische Bezeichnung eine geringere Krümmung aufweist, und
wobei die zweite medizinische Bezeichnung eine stärkere Krümmung aufweist.

3. Verfahren zum Betätigen einer Vorrichtung zum Einstellen von Koordinaten jedes Teils eines Organs eines menschlichen Körpers durch eine Isolinie in der Chirurgie, wobei das Verfahren umfasst:
Empfangen von Organinformationen über das Organ, wobei die Organinformationen ein Organbild (300) enthalten, welches das Organ (310) darstellt;
Festlegen einer Vielzahl von Isolinien (EPL1, EPL2, EPLn-1, EPLn), die einen ersten Bereich (410), der einem das Organ bildenden ersten Teil entspricht, und einen zweiten Bereich (420), der einem das Organ bildenden zweiten Teil entspricht, verbinden, wobei die Vielzahl von Isolinien (EPL1, EPL2, EPLn-1, EPLN) auf einer Innenumfangsfläche positioniert sind, die einer Peripherie des Organs entspricht; und Erzeugen von Organ-Koordinatensystem-Informationen, welche die Organinformationen und Isolinieninformationen über die Vielzahl von Isolinien (EPL1, EPL2, EPLN- 1, EPLN) enthalten,
wobei das Festlegen der Vielzahl von Isolinien umfasst: Festlegen einer Vielzahl von ersten Punkten (P11, P12, Pin-1, Pin) auf dem ersten Bereich (410); Festlegen einer Vielzahl von zweiten Punkten (P21, P22, P2n-1, P2n) auf dem zweiten Bereich (420); und Festlegen der Vielzahl von Isolinien (EPL1, EPL2, EPLn-1, EPLn) durch Verbinden der Vielzahl von ersten Punkten (P11, P12, Pin-1, Pin) und der Vielzahl von zweiten Punkten (P21, P22, P2n-1, P2n), die in der gleichen Reihenfolge von Startpunkten zu Endpunkten des ersten Bereichs (410) und des zweiten Bereichs (420) positioniert sind,
**dadurch gekennzeichnet, dass** die Vielzahl von ersten Punkten (P11, P12, Pin-1, Pin) in gleichen Abständen festgelegt werden,
wobei die Vielzahl von zweiten Punkten (P21, P22, P2n-1, P2n) in gleichen Abständen festgelegt werden,
wobei jeder der Vielzahl von Isolinien (EPL1, EPL2, EPLn-1, EPLN) eine Nummer zugeordnet wird, und zwar nacheinander von einer Isolinie, die durch Verbinden der Startpunkte gebildet wird, bis zu einer Isolinie, die durch Verbinden der Endpunkte gebildet wird, und
wobei das Verfahren ferner umfasst: Berechnen eines Abstands, der von der Vielzahl von ersten Punkten (P11, P12, Pin-1, P1n) oder der Vielzahl von zweiten Punkten (P21, P22, P2n-1, P2n), die in der gleichen Reihenfolge positioniert sind, auf einer Isolinie beabstandet ist, die durch Verbinden der Vielzahl von ersten Punkten und der Vielzahl von zweiten Punkten, die in der gleichen Reihenfolge positioniert sind, gebildet wird; Erfassen einer Vielzahl von dritten Punkten (P31, P32, P3n-1, P3n), die in dem Abstand von der Vielzahl von ersten Punkten oder der Vielzahl von zweiten Punkten, die in der gleichen Reihenfolge positioniert sind, positioniert sind; und Erzeugen der Organ-Koordinatensystem-Informationen, die ferner Punktinformationen enthalten, welche die Vielzahl von ersten Punkten (P11, P12, P1n-1, P1n), die Vielzahl von zweiten Punkten (P21, P22, P2n-1, P2n) und die Vielzahl von dritten Punkten (P31, P32, P3n-1, P3n) darstellen.

4. Verfahren nach Anspruch 3, wobei die Organinformationen eine Bezeichnung des Organs (310) und medizinische Bezeichnungen der jeweiligen Teile, aus denen das Organ (310) besteht, enthalten,
wobei der erste Teil einer ersten medizinischen Bezeichnung entspricht,
wobei der zweite Teil einer zweiten medizinischen Bezeichnung entspricht,
wobei das Organ einem Magen entspricht,
wobei die erste medizinische Bezeichnung eine geringere Krümmung aufweist, und
wobei die zweite medizinische Bezeichnung eine stärkere Krümmung aufweist.

5. Computerlesbares Aufzeichnungsmedium, das mit einem Computer gekoppelt ist und ein Programm zum Ausführen des Verfahrens nach einem der Ansprüche 3 bis 4 speichert.

## Revendications

1. Dispositif d'établissement de coordonnées de chaque partie d'un organe d'un corps humain par l'intermédiaire d'une isoligne lors d'une chirurgie, le dispositif comprenant :
une mémoire (110) ; et
un processeur (120) configuré pour recevoir des informations d'organe concernant l'organe, dans lequel les informations d'organe comprennent une image d'organe (300) représentant l'organe (310), pour définir une pluralité d'isolignes (EPL1, EPL2, EPLn-1, EPLn) reliant une première zone (410) correspondant à une première partie constituant l'organe et une seconde zone (420) correspondant à une seconde partie constituant l'organe, dans lequel les isolignes de la pluralité d'isolignes (EPL1, EPL2, EPLN-1, EPLn) sont positionnées sur une surface circonférentielle interne correspondant à une périphérie de l'organe, et pour générer des informations de système de coordonnées d'organe comprenant des informations d'isolignes représentant les isolignes de la pluralité d'isolignes (EPL1, EPL2, EPLn-1, EPLn) et les informations d'organe,
dans lequel le processeur (120) est configuré pour : définir une pluralité de premiers points (P11, P12, Pin-1, Pin) sur la première zone (410) ; et pour définir une pluralité de deuxièmes points (P21, P22, P2n-1, P2n) sur la seconde zone (420), et
dans lequel les isolignes de la pluralité d'isolignes (EPL1, EPL2, EPLn-1, EPLn) sont définies en reliant les premiers points de la pluralité de premiers points (P11, P12, Pin-1, Pin) et les deuxièmes points de la pluralité de deuxièmes points (P21, P22, P2n-1, P2n) positionnés selon le même ordre de points de début à points de fin de la première zone (410) et de la seconde zone (420),
**caractérisé en ce que** le processeur (120) est configuré pour : définir les premiers points de la pluralité de premiers points (P11, P12, Pin-1, Pin) à des intervalles égaux ; définir les points de la pluralité de deuxièmes points (P21, P22, P2n-1, P2n) à des intervalles égaux,
dans lequel un numéro est défini successivement pour chaque isoligne de la pluralité d'isolignes (EPL1, EPL2, EPLn-1, EPLN) à partir d'une isoligne formée en reliant les points de début à une isoligne formée en reliant les points de fin, et
dans lequel le processeur (120) est en outre configuré pour : calculer une distance d'espacement des points de la pluralité de premiers points (P11, P12, Pin-1, Pin) ou les points de la pluralité de deuxièmes points (P21, P22, P2n-1, P2n) positionnés selon le même ordre sur une isoligne formée en reliant les points de la pluralité de premiers points et les points de la pluralité de deuxièmes points positionnés selon le même ordre ; acquérir une pluralité de troisièmes points (P31, P32, P3n-1, P3n) positionnés à la distance d'espacement des points de la pluralité de premiers points ou des points de la pluralité de deuxièmes points positionnés selon le même ordre ; et générer les informations de système de coordonnées d'organe comprenant en outre des informations de points représentant les points de la pluralité de premiers points (P11, P12, P1n-1, P1n), les points de la pluralité de deuxièmes points (P21, P22, P2n-1, P2n) et les points de la pluralité de troisièmes points (P31, P32, P3n-1, P3n).

2. Dispositif selon la revendication 1,
dans lequel les informations d'organe comprennent un nom de l'organe (310) et des noms médicaux de parties respectives constituant l'organe (310),
dans lequel la première partie correspond à un premier nom médical,
dans lequel la seconde partie correspond à un second nom médical,
dans lequel l'organe correspond à un estomac,
dans lequel le premier nom médical comprend une petite courbure, et
dans lequel le second nom médical comprend une grande courbure.

3. Procédé de mise en œuvre d'un dispositif d'établissement de coordonnées de chaque partie d'un organe d'un corps humain par l'intermédiaire d'une isoligne lors d'une chirurgie,
le procédé comprenant les étapes consistant à :
recevoir des informations d'organe concernant l'organe, dans lequel les informations d'organe comprennent une image d'organe (300) représentant l'organe (310) ;
définir une pluralité d'isolignes (EPL1, EPL2, EPLn-1, EPLn) reliant une première zone (410) correspondant à une première partie constituant l'organe et une seconde zone (420) correspondant à une seconde partie constituant l'organe, dans lequel les isolignes de la pluralité d'isolignes (EPL1, EPL2, EPLN-1, EPLN) sont positionnées sur une surface circonférentielle interne correspondant à une périphérie de l'organe ; et
générer des informations de système de coordonnées d'organe comprenant les informations d'organe et les informations d'isolignes des isolignes de la pluralité d'isolignes (EPL1, EPL2, EPLN-1, EPLN),
dans lequel l'étape de définition des isolignes de la pluralité d'isolignes consiste à : définir une pluralité de premiers points (P11, P12, Pin-1, Pin) sur la première zone (410) ; définir une pluralité de deuxièmes points (P21, P22, P2n-1, P2n) sur la seconde zone (420) ; et définir la pluralité d'isolignes (EPL1, EPL2, EPLn-1, EPLn) en reliant les premiers points de la pluralité de premiers points (P11, P12, Pin-1, Pin) et les deuxièmes points de la pluralité de deuxièmes points (P21, P22, P2n-1, P2n) positionnés selon le même ordre de points de début à points de fin de la première zone (410) et de la seconde zone (420),
**caractérisé en ce que** les points de la pluralité de premiers points (P11, P12, Pin-1, Pin) sont définis à des intervalles égaux,
dans lequel les points de la pluralité de deuxièmes points (P21, P22, P2n-1, P2n) sont définis à des intervalles égaux,
dans lequel un numéro est défini successivement pour chaque isoligne de la pluralité d'isolignes (EPL1, EPL2, EPLn-1, EPLN) à partir d'une isoligne formée en reliant les points de début à une isoligne formée en reliant les points de fin, et
dans lequel le procédé comprend en outre les étapes consistant à : calculer une distance d'espacement des points de la pluralité de premiers points (P11, P12, Pin-1, Pin) ou des points de la pluralité de deuxièmes points (P21, P22, P2n-1, P2n) positionnés selon le même ordre sur une isoligne formée en reliant les points de la pluralité de premiers points et les points de la pluralité de deuxièmes points positionnés selon le même ordre ; acquérir une pluralité de troisièmes points (P31, P32, P3n-1, P3n) positionnés à la distance d'espacement des points de la pluralité de premiers points ou des points de la pluralité de deuxièmes points positionnés selon le même ordre ; et générer les informations de système de coordonnées d'organe comprenant en outre des informations de points représentant les points de la pluralité de premiers points (P11, P12, Pin-1, Pin), les points de la pluralité de deuxièmes points (P21, P22, P2n-1, P2n) et les points de la pluralité de troisièmes points (P31, P32, P3n-1, P3n).

4. Procédé selon la revendication 3,
dans lequel les informations d'organe comprennent un nom de l'organe (310) et des noms médicaux de parties respectives constituant l'organe (310),
dans lequel la première partie correspond à un premier nom médical,
dans lequel la seconde partie correspond à un second nom médical,
dans lequel l'organe correspond à un estomac,
dans lequel le premier nom médical comprend une petite courbure, et
dans lequel le second nom médical comprend une grande courbure.

5. Support d'informations lisible par ordinateur couplé à un ordinateur et maintenant un programme permettant d'exécuter le procédé selon l'une ou l'autre des revendications 3 et 4.
